# EUROPEAN PATENT SPECIFICATION

(11) **EP 3 626 235 B1**
(45) Date of publication and mention of the grant of the patent: **13.12.2023**
(21) Application number: 19206658.7
(22) Date of filing: 24.09.2015
(51) Int. Cl.: A61K 31/05, A61K 31/12, A61K 31/202, A61P 35/00, A61P 29/00

(54) **PHARMACEUTICAL PRODUCT, MEDICAL FOOD OR DIETARY SUPPLEMENT FOR PREVENTING CANCER AND INFLAMMATORY DISEASES**
PHARMAZEUTISCHES PRODUKT, MEDIZINISCHES NAHRUNGSMITTEL ODER NAHRUNGSZUSATZ ZUR VERHINDERUNG VON KREBS UND ENTZÜNDUNGSKRANKHEITEN
PRODUIT PHARMACEUTIQUE, ALIMENT MÉDICAL OU SUPPLÉMENT DIÉTÉTIQUE POUR LA PRÉVENTION DU CANCER ET DE MALADIES INFLAMMATOIRES

(30) Priority: 24.09.2014 EP 14382357; 17.10.2014 US 201414516906
(43) Date of publication of application: 25.03.2020
(62) Divisional of application: 15778228.5
(73) Proprietor: Phytogen Medical Foods S.L., 28009 Madrid (ES)
(72) Inventor: Peña Díaz, Carlos María, 28009 Madrid (ES); Muñoz Fernández, Guillermo, 28009 Madrid (ES); Morse, Michael, 28009 Madrid (ES)
(74) Representative: Hoffmann Eitle

(56) References cited:
- WO-A1-2006/020588
- WO-A2-2012/037328
- US-A1- 2004 146 551
- SUBASH C GUPTA ET AL: "Therapeutic Roles of Curcumin: Lessons Learned from Clinical Trials", AAPS JOURNAL, AMERICAN ASSOCIATION OF PHARMACEUTICAL SCIENTISTS, US, vol. 15, no. 1, 10 November 2012 (2012-11-10), pages 195-218, XP035156222, ISSN: 1550-7416, DOI: 10.1208/S12248-012-9432-8
- JUNKYU HAN ET AL: "Anti-proliferative and apoptotic effects of oleuropein and hydroxytyrosol on human breast cancer MCF-7 cells", CYTOTECHNOLOGY, KLUWER ACADEMIC PUBLISHERS, DO, vol. 59, no. 1, 8 April 2009 (2009-04-08), pages 45-53, XP019673342, ISSN: 1573-0778
- GARG A K: "Fatigue, Inflammation, and [omega]-3 and [omega]-6 Fatty Acid Intake Among Breast Cancer Survivors Alfano CM, Imayama I, Neuhouser ML, et al (Natl Cancer Inst, Bethesda, MD; Fred Hutchinson Cancer Res Ctr, Seattle, WA; et al)J Clin Oncol30:1280-1287, 2012$", BREAST DISEASES: A YEAR BOOK QUARTERLY, MOSBY, ST. LOUIS, MO, US, vol. 24, no. 1, 15 March 2013 (2013-03-15) , pages 33-34, XP029001788, ISSN: 1043-321X, DOI: 10.1016/J.BREASTDIS.2013.01.015
- SCHLEY PATRICIA D ET AL: "Eicosapentaenoic acid and docosahexaenoic acid induce apoptosis in MDA-MB-231 and MCF-7 human breast cancer cells.", FASEB JOURNAL, vol. 17, no. 4-5, March 2003 (2003-03), XP008175288, & FASEB MEETING ON EXPERIMENTAL BIOLOGY: TRANSLATING THE GENOME; SAN DIEGO, CA, USA; APRIL 11-15, 2003 ISSN: 0892-6638
- HUTCHINS-WIESE HEATHER L ET AL: "High-dose eicosapentaenoic acid and docosahexaenoic acid supplementation reduces bone resorption in postmenopausal breast cancer survivors on aromatase inhibitors: a pilot study", NUTRITION AND CANCER ENGLAND 2010, TAYLOR & FRANCIS, US, vol. 66, no. 1, 1 January 2014 (2014-01-01), pages 68-76, XP008178190, ISSN: 1532-7914, DOI: 10.1080/01635581.2014.847964 [retrieved on 2013-11-25]
- CATHERINE M. ALFANO ET AL: "Fatigue, Inflammation, and omega-3 and omega-6 Fatty Acid Intake Among Breast Cancer Survivors", JOURNAL OF CLINICAL ONCOLOGY, vol. 30, no. 12, 20 April 2012 (2012-04-20), pages 1280-1287, XP055594651, US ISSN: 0732-183X, DOI: 10.1200/JCO.2011.36.4109

## Description

### Field of the invention

The present invention relates to cancer therapy by administering a specific dietary compensation. Especially the invention relates to a pharmaceutical, medical food or dietary supplement composition comprising the combination of the following three active ingredients: hydroxytyrosol, fish oil EPA/DHA and curcumin. The pharmaceutical composition is useful in the treatment or prevention of cancer, especially breast cancer.

### Background of the invention

There is growing evidence that chronic inflammation plays an important role in the development of human cancer. Several chronic inflammatory processes have been clearly associated with specific cancers, such as Crohn's disease and chronic ulcerative colitis with colorectal cancer, chronic bronchitis with lung cancer, and chronic pancreatitis with pancreatic cancer. The inflammatory component of chronic infections is a key element in the carcinogenic risk among carriers, e.g., of liver cancer among hepatitis B carriers and cholangiocarcinoma among individuals with liver fluke infestation. The unspecific nature of the role of chronic inflammation in human carcinogenesis is substantiated by the observation of a reduced risk of several types of cancer with use of aspirin and anti-inflammatory agents.

C-reactive protein (CRP) is produced by the liver and other organs in response to release of interleukin-6 by monocytes and other immune cells following infection and other conditions associated with tissue injury and inflammation. Elevated levels of this marker of inflammation have been associated with increased risk of cardiovascular disease, as well as of increased overall mortality in the elderly. A few studies have been recently published on the association between CRP level and cancer risk. In this sense, one of these studies used a prospective cohort study of 2,910 Danish women with invasive breast cancer, and demonstrated that elevated CRP levels at the time of diagnosis of breast cancer were associated with reduced overall and disease-free survival and with increased risk of death from breast cancer.

Mechanistically, three components might explain the observed association between elevated CRP levels and poor breast cancer prognosis. First, tumour cell behaviour: plasma CRP levels may reflect the aggressiveness of the tumour, that is, plasma CRP levels might sum up some prognostic information of well-known tumour characteristics, such as tumour stage and grade. In fact, in the Danish study elevated CRP levels were indeed associated with larger tumour size, presence of distant metastases, and lower tumour grade (although CRP was not linearly associated with tumour grade), and these prognostic factors were associated with poor prognosis. Second, adjacent inflammation: plasma CRP levels might express the magnitude and the nature of any inflammation in the breast tumour microenvironment. Inflammatory pathways play important roles in all stages of tumourigenesis, including tumour initiation and promotion, malignant transformation, tumour invasion, and metastasis. Thus, solid tumours typically trigger inflammatory responses that result in the formation of a pro-tumourigenic and pro-angiogenic microenvironment around the tumour. Immune and inflammatory cells in the tumour microenvironment interact with malignant cells in a complicated fashion, the net result of which is stimulation of tumour growth, invasion, and metastasis. Despite the fact that breast cancers rarely are characterized by significant histological inflammation, inflammation might also play a role in breast cancer prognosis. Thus, macrophage infiltration into invasive breast carcinomas was associated with high vascularity of the breast tumour as well as with reduced recurrence-free and overall survival, and targeting of cancer associated fibroblasts resulted in favourable changes of the immune tumour microenvironment and improved anti-metastatic effects of doxorubicin chemotherapy in a murine model of metastatic breast cancer. Furthermore, a recently published study showed that blockade of the IL-8 receptor selectively targets breast cancer stem cells and retards tumour growth and reduces metastasis. Third, host behaviour: plasma CRP levels may outline the general health of the woman at the time of diagnosis of breast cancer.

Therefore there is a positive association between elevated CRP levels and poor breast cancer prognosis. In fact, elevated CRP levels are associated with reduced overall survival irrespective of age at diagnosis, tumour size, lymph node status, presence of distant metastases, tumour grade, and estrogen receptor, progesterone receptor, and HER2 status. Furthermore, it has been established that by dividing plasma CRP levels into octiles resulted in a stepwise increased risk of reduced overall survival, demonstrating the robustness of the observed association between elevated CRP levels and risk of reduced overall survival. Furthermore, it has been observed that compared to women with CRP levels in the 0 to 25% percentile (CRP <0.78 mg/L), women with CRP levels ≥95% percentile (≥16.4 mg/L) had a 3.5-fold increased risk of reduced overall survival. Moreover, among women with HER2-positive tumours, there is a 8.63 fold reduced overall survival for the highest versus the lowest tertile, concluding that women with high CRP levels at the time of diagnosis have a particularly poor survival.

Based on the previously mentioned results, there is a need to investigate potential anti-inflammatory and anti-cancerous products capable of reducing one or more octiles the plasma CRP levels.

In this sense, there are contradictory results whether the regular use of fish oil supplements is associated with lower CRP concentrations. Fish oil contains long-chain omega-3 polyunsaturated fatty acids (PUFAs), such as eicosapentaenoic acid and docosahexaenoic acid. These omega-3 PUFAs are thought to reduce inflammation in several ways, including inhibition of nuclear factor kappa B activation and competitive inhibition of pro-inflammatory omega-6 PUFAs. Omega-3 PUFAs compete with omega-6 PUFAs for the cyclooxygenase 2 enzyme and displace omega-6 stores in cell membranes. There have been numerous human trials of omega-3 supplements and CRP or other markers of inflammation, primarily small trials of subjects at high risk of cardiovascular disease. Two reviews published in 2006 concluded that the trials were inconsistent and inconclusive (Balk EM, Lichtenstein AH, Chung M, et al. Effects of omega-3 fatty acids on serum markers of cardiovascular disease risk: a systematic review. Atherosclerosis 2006;189(1):19-30 I.F.: 3.908 and Fritsche K. Fatty acids as modulators of the immune response. Annu Rev Nutr 2006;26:45-73). I.F: 8.2).

More recently, however, 2 randomized controlled trials of omega-3 supplementation found that the supplements reduced circulating CRP (Ebrahimi M, Ghayour-Mobarhan M, Rezaiean S, et al. Omega-3 fatty acid supplements improve the cardiovascular risk profile of subjects with metabolic syndrome, including markers of inflammation and auto-immunity. Acta Cardiol 2009;64(3):321-327 I.F.: 0.604 and Micallef MA, Garg ML. Anti-inflammatory and cardioprotective effects of n-3 polyunsaturated fatty acids and plant sterols in hyperlipidemic individuals (in this study the reduction of CRP was observed with fatty acids together with plant sterols) Atherosclerosis 2009;204(2):476-482) and tumour necrosis factor alpha levels. These studies suggests an evidence for the anti-inflammatory effects of long-chain omega-3 PUFAs in humans, and they support one of several mechanisms by which long-chain omega-3 PUFA intake may reduce the risk of cardiovascular disease, some cancers, and total mortality.

In conclusion, there is inconsistency between all the studies and reviews published, whether omega-3 fatty acids provide a significant reduction of CRP plasma levels.

ALFANO CATHERINE M ET AL: "Fatigue, inflammation, and [omega]-3 and [omega]-6 fatty acid intake among breast cancer survivors.", JOURNAL OF CLINICAL ONCOLOGY : OFFICIAL JOURNAL OF THE AMERICAN SOCIETY OF CLINICAL ONCOLOGY 20 APR 2012, vol. 30, no. 12, 20 April 2012 (2012-04-20), pages 1280-1287, discloses that breast cancer survivors with the lowest ω-6:ω-3 ratio who were taking ω-3 supplements had the lowest serum CRP levels and greater odd of fatigue.

Thus, despite the research efforts to find a pharmaceutical, medical food or dietary supplement capable of increasing the overall survival of cancer patients, particularly of breast cancer patients, there is still a need to find such an agent that is capable of increasing the overall survival of cancer patients by reducing one or more octiles the plasma CRP levels of such patients.

### Brief Description of the invention

The references to methods of treatment in the brief and detailed description of the invention in this description are to be interpreted as references to the compounds, pharmaceutical compositions and medicaments of the present invention for use in a method of treatment of the human or animal body by therapy.

The authors of the present invention have found a product (pharmaceutical, medical food or dietary supplement) capable of significantly reducing, in patients who have previously had surgical resection of breast cancer, the plasma levels of CRP, a biomarker of inflammation positively associated with reduced overall survival of patients with breast cancer. Such composition comprises (from hereinafter referred to as "composition of the invention" or "investigational product") the following active ingredients in the following amounts:
a. An amount of +/-30% of from 243.8 mg to 731.4 mg of Omega-3 polyunsaturated fatty acids (PUFAs) EPA and DHA, in a weight ratio of EPA:DHA of from 1.2 to 1.8;
b. An amount of +/-30% of from 12.5 mg to 37.5 mg of hydroxytyrosol; and
c. An amount of +/-30% of from 40 mg to 120 mg of curcumin.

The composition of the present invention can be used as a pharmaceutical, medical food or dietary supplement composition, optionally comprising pharmaceutical or nutraceutical acceptable excipients.

The composition of the invention is especially suitable in a method of reducing CRP in plasma. In particular, the composition of the invention is especially suitable in a method of reducing CRP in plasma when said composition is administered in one or more daily dosages so that the daily amount of each of the three components is +/-30% of 731.4 mg of Omega-3 polyunsaturated fatty acids (PUFAs) EPA and DHA, +/-30% of 37.5 mg of hydroxytyrosol and +/-30% of 120 mg of curcumin and wherein said composition is administered orally.

Lastly, the composition of the invention is especially suitable in a method of increasing the overall survival rate of breast cancer patients diagnosed with said disease. In particular, the composition of the invention is especially suitable in a method of increasing the overall survival rate of breast cancer patients diagnosed with said disease, when said composition is administered in one or more daily dosages so that the daily amount of each of the three components is +/-30% of 731.4 mg of Omega-3 polyunsaturated fatty acids (PUFAs) EPA and DHA, +/-30% of 37.5 mg of hydroxytyrosol and +/-30% of 120 mg of curcumin, and wherein said composition is administered orally.

### Brief description of the figures

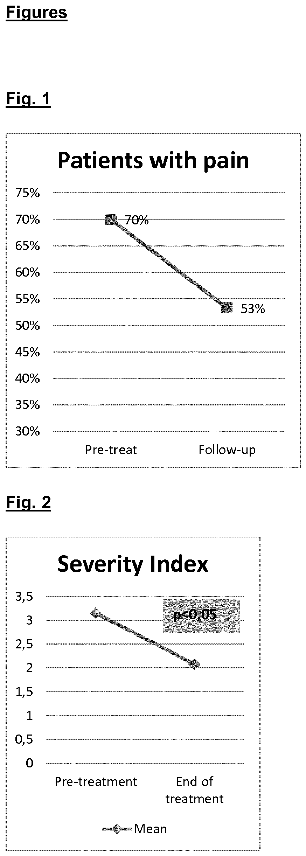

Both figures 1 and 2 show the results directed to the secondary variable: scores of mean pain intensity with stable administration measured with the BPI scale, obtained in 30 out of the 32 woman with stage 0-IIIA breast cancer participating in the clinical trial herein reported.
Figure 1 shows a statistical significant decrease of the patients that reported pain before the treatment after treatment with the investigational product.
Figure 2 shows a statistically significant decrease in the severity of the pain reported by the patients after treatment.

### Detailed description of the invention

The present invention provides an inexpensive and safe pharmaceutical, medical food or dietary supplement composition comprising natural, biological components for reducing CRP levels in plasma. In this sense, it has now surprisingly been found that a product which comprises the combination of the following three active ingredients: hydroxytyrosol, fish oil EPA/DHA and curcumin is capable of reducing CRP levels in plasma (on average) in more than 2.0 mg/L in human subjects.

In this sense, the authors of the present invention have conducted a clinical pilot study in human subjects to determine changes in certain inflammatory markers in women diagnosed with breast cancer (see examples for the clinical study protocol).

In order to undertake the previously mentioned clinical study, the authors of the present invention administered a pharmaceutical, medical food or dietary supplement composition (from hereinafter "composition of the invention" or "investigational product") two times per day (three capsules) comprising the following active ingredients per capsule:

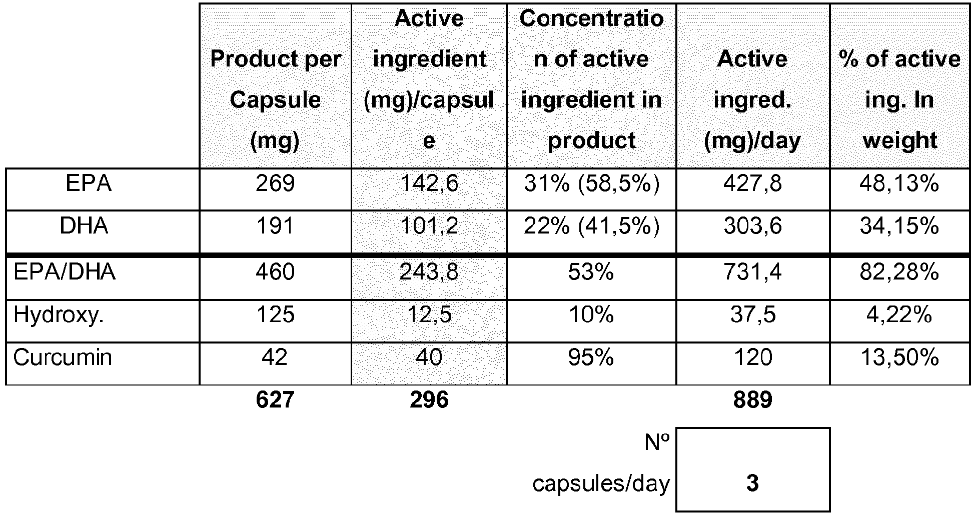

In particular, the patients were administered three capsules per day of the following pharmaceutical, medical food or dietary supplement composition per capsule (including active ingredients and excipients):

| | **mg/capsule** | **g/100g** |
|---|---|---|
| Fish oil (Triglycerid form) 310 mg/g EPA and 220 mg/g DHA (ONC) | 460 | 55 |
| Hytolive 10% powder | 125 | 15 |
| Gelatin 98. | 9 | 12 |
| Mono and diglycerides of fatty acids (E471) | 50.0 | 6.0 |
| Curcumin Powder 95% | 42.0 | 5.0 |
| Soybean oil, refined | 28.0 | 3.3 |
| Water | 16.8 | 2.0 |
| Soybean lecithin solubilized in Soya | | |
| oil,enriched with phosphatidylcholine | 15.0 | 1.8 |
| Iron Oxide (E172) | 1.77 | 0.21 |
| Titanium dioxide (E171) | 0.590 | 0.070 |

As used herein, the term "Hytolive" is understood as a natural extract from olive fruit with a high purity in natural hydroxytyrosol. In particular, hytolive refers to a composition comprising the following ingredients:

| | |
|---|---|
| Carrier (Maltodextrins) | 40,0-60,0% |
| Hydroxytyrosol | 10,0-20,0% |
| Ash | 1,0-8,0% |
| Other Phenolics | 2,0-5,0% |
| Anticaking agent (SiO₂) | 0,1-2,0% |
| Water | 0,1-3,0% |
| Flavonoids | 0,1-1,0% |
| Other plant material | Rest up to 100% |

| | |
|---|---|
| Carrier (Maltodextrins) | 40,0-60,0% |
| Hydroxytyrosol | 10,0-20,0% |
| Ash | 1,0-8,0% |
| Other Phenolics | 2,0-5,0% |
| Anticaking agent (SiO₂) | 0,1-2,0% |
| Water | 0,1-3,0% |
| Flavonoids | 0,1-1,0% |
| Other plant material | Rest up to 100% |

Hytolive was acquired by the inventors from Genosa I+D S.A under product name Hytolive^{®} Powder and product code 40610. Hytolive is an olive fruit extract manufactured from the vegetable water generated during the olive oil extraction (see patent application PCT/ES02/00058). This water is physically filtered and evaporated. The concentrated vegetable water is subjected to ion exchange column system containing a food grade anion exchange resin to obtain hydroxytyrosol syrup. The chromatographic column with anion exchange resin primarily retains hydroxytyrosol, tyrosol and organic acids based on the polarity of these compounds. For elution demineralized water is used. The water phase following elution is concentrated by evaporation, sterilized to obtain syrup (hytolive).

In order to prepare hytolive in powder form, food grade vegetable carrier (maltodextrin) and silicon dioxide is thoroughly mixed with the syrup obtained from the above steps. The mixture is dried, leading to the formation of powder. The whole extraction process is performed without solvents.

As used herein, "Soybean lecithin solubilized in Soya oil, enriched with phosphatidylcholine" is understood as one of the possible excipients to be used in the formulation of the product. Other excipients approved for pharmaceutical, medical foods, or dietary supplements composition could also be used.

The clinical study followed the principles outline in the Declaration of Helsinki and was approved by the local ethics committees. Each of the patients gave full informed consent. The clinical study was a multicentric, one-label cohort study carried out in breast-cancer patients free of disease in the past 24 months.

Inclusion criteria were:
- Post-menopause women with history of 0-IIIA stage breast cancer (according to the American Joint Committee on Cancer, AJCC) surgically resected in the previous 2 to 5 years;
- Stable aromatase inhibitor (letrozol, anastrozol, exemestane) or Tamoxifen therapy for at least three month before the beginning of the study;
- Serum C-reactive protein (CRP) ≥3.9 as quantified by the average of two consecutive analyses; and
- No chemotherapy for at least the previous six months; willingness to complete the study.

Exclusion criteria were:
- Cancer other than breast cancer;
- Cardiovascular or autoimmune disease;
- Use of corticosteroids or immunosuppressors; immunodeficiency, e.g. HIV;
- Habitual use of aspiring > 91 mg/d or FANS >400 mg 4 times/d or other COX-2 inhibitors;
- Use of bisfosfonates; and
- Use of supplements, extra virgin olive oil, and olives during the previous month and throughout the study.

Blood samples were drawn in vacutainers before and after administration of the composition of the invention. After centrifugation, serum was separated, aliquoted, and stored at -80 °C. In addition routine hemochrome and plasma lipids determinations were performed.

The beginning of treatment started no later than 28 days after the date of the first extraction of the selection period. Therefore, the treatment with the investigational product began on day 0 of the trial. It is noted that, to date, the investigational product is not available to the public and was only administered to the patients once they provided their full consent to the conditions of the trial.

On day 14 of treatment each patient was evaluated, in this sense a clinical history was made and they were questioned on adverse events or toxicity related to the administration of the product.

At the end-of-treatment visit, on day 30, a clinical history was made again. By that time, two further extractions were performed, on day 30 and day 33 (+/- 2 days), with 10 mL of blood taken per extraction that were processed as described above.

Finally, on day 60 from day 0 of the trial, the patients were asked about their general condition and whether they suffered any adverse events, related or not to the medication. At that time, one further extraction was performed, with 10 mL of blood taken that was processed as described above.

The results on day 30 of treatment, associated to the CRP determination per patient in the 32 patients, are shown in each of the rows of table I below (the concentrations below are expressed in mg/L):

**Table I**

| Average Pre-treatment (CRP1) | Average post-treatment (CRP2) | Avg CRP2-Avg CRP1(Value) | %CRP Variation PRE-POST |
|---|---|---|---|
| 9 | 1,05 | -7,95 | -88% |
| 5,4 | 0,80 | -4,60 | -85% |
| 8,035 | 1,44 | -6,60 | -82% |
| 12,55 | 2,25 | -10,30 | -82% |
| 19 | 6,00 | -13,01 | -68% |
| 6,315 | 2,18 | -4,14 | -66% |
| 4,95 | 2,00 | -2,95 | -60% |
| 5,85 | 2,75 | -3,11 | -53% |
| 5,195 | 2,85 | -2,35 | -45% |
| 7,45 | 4,40 | -3,05 | -41% |
| 4,985 | 3,15 | -1,84 | -37% |
| 16,085 | 10,45 | -5,64 | -35% |
| 8,95 | 5,90 | -3,05 | -34% |
| 4,15 | 2,95 | -1,20 | -29% |
| 4,1 | 2,95 | -1,15 | -28% |
| 5,505 | 3,99 | -1,52 | -28% |
| 6,105 | 4,85 | -1,26 | -21% |
| 5,995 | 5,05 | -0,94 | -16% |
| 10,95 | 9,70 | -1,25 | -11% |
| 6 | 5,35 | -0,65 | -11% |
| 5,42 | 5,35 | -0,07 | -1% |
| 11,895 | 11,75 | -0,15 | -1% |
| 4,775 | 4,85 | 0,07 | 2% |
| 4,8 | 4,95 | 0,15 | 3% |
| 6,3 | 6,55 | 0,25 | 4% |
| 5,5 | 6,25 | 0,75 | 14% |
| 7,095 | 8,77 | 1,67 | 24% |
| 4,155 | 5,24 | 1,09 | 26% |
| 3,92 | 5,05 | 1,13 | 29% |
| 4,18 | 6,23 | 2,05 | 49% |
| 4,635 | 7,31 | 2,67 | 58% |
| 5,165 | 15,26 | 10,10 | 195% |

As shown in table 1 above, surprisingly, 69% of the patients included in the clinical trial reduced their CRP levels in plasma. In particular and within the 69% group of patients having reduced CRP levels in plasma, the average reduction was approximately 3.49 mg/L (42% CRP reduction in plasma). This is an outstanding reduction if we take into account that compared to women with CRP levels in the 0 to 25% percentile (CRP <0.78 mg/L), women with CRP levels ≥95% percentile (≥16.4 mg/L) had a 3.5-fold increased risk of reduced overall survival. In this sense, table I above shows that most of the patients reduced their CRP levels in plasma in one or more octiles (octiles are detailed in table 2 below), thus decreasing the risk of reduced overall survival.

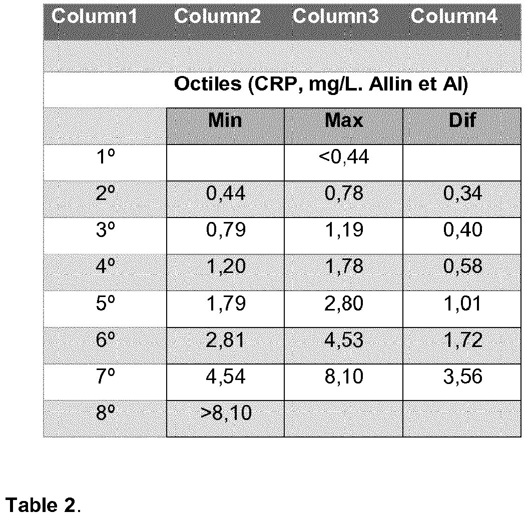

In addition we herein show the results after two months of the initial treatment, associated to the CRP determination per patient in 27 patients (the concentrations below are expressed in mg/L):

**Table 3**

| Average Prestreatment | CRP Day+60 | CRP Variation PRE- D+60 | % CRP Variation PRE- D+60 |
|---|---|---|---|
| 9 | 1,2 | -7,8 | -87% |
| 8,035 | 1,57 | -6,465 | -80% |
| 12,55 | 2,9 | -9,65 | -77% |
| 19 | 5,4 | -13,6 | -72% |
| 5,995 | 1,88 | -4,115 | -69% |
| 6,315 | 2,15 | -4,165 | -66% |
| 7,095 | 3,3 | -3,795 | -53% |
| 5,85 | 2,97 | -2,88 | -49% |
| 4,8 | 2,5 | -2,3 | -48% |
| 4,95 | 2,6 | -2,35 | -47% |
| 6 | 3,2 | -2,8 | -47% |
| 10,95 | 6,2 | -4,75 | -43% |
| 16,085 | 9,3 | -6,785 | -42% |
| 4,1 | 2,4 | -1,7 | -41% |
| 6,3 | 4,1 | -2,2 | -35% |
| 5,4 | 3,6 | -1,8 | -33% |
| 8,95 | 6,7 | -2,25 | -25% |
| 5,505 | 4,22 | -1,285 | -23% |
| 5,195 | 5,11 | -0,085 | -2% |
| 5,165 | 5,53 | 0,365 | 7% |
| 3,92 | 4,36 | 0,44 | 11% |
| 5,42 | 6,61 | 1,19 | 22% |
| 4,775 | 6,38 | 1,605 | 34% |
| 4,18 | 5,8 | 1,62 | 39% |
| 4,155 | 6,12 | 1,965 | 47% |
| 6,105 | 15,4 | 9,295 | 152% |
| 4,985 | 20,9 | 15,915 | 319% |

As shown in table 3 above, surprisingly, one month after stopping the treatment still 70% of the patients included in the clinical trial reduced their CRP levels in plasma. In particular and within the 70% group of patients having reduced CRP levels in plasma, the average reduction was approximately 2.54 mg/L (24% CRP reduction in plasma) after 60 days. This is an outstanding reduction if we take into account that no further treatment was administered to the patients after one month of initiating the study. In this sense, table 3 above shows that the investigational product bears a long lasting effect in most of the patients, in which we still find significantly reduced CRP levels in plasma after 30 days of having stopped the treatment.

The present invention thus provides for a composition, particularly suitable for efficiently reducing CRP levels in plasma. In particular, as shown, the pharmaceutical, medical food or dietary supplement of the present invention is capable of increasing the overall survival of cancer patients, in particular of breast cancer patients, by reducing one or more octiles the plasma CRP levels of such patients. The invention further provides a composition for use as a medicament, and especially for use in the treatment or prevention of cancer, preferably prostate, breast or cervix cancer.

### -The pharmaceutical, medical food or dietary supplement composition of the invention

The present invention provides for a composition, preferably in the form of a medical food, dietary supplement composition or of a pharmaceutical composition, which comprises at least the following active ingredients in the following amounts:
a. An amount of +/-30% of from 243.8 mg to 731.4 mg of Omega-3 polyunsaturated fatty acids (PUFAs) EPA and DHA, in a weight ratio of EPA:DHA of from 1.2 to 1.8;
b. An amount of +/-30% of from 12.5 mg to 37.5 mg of hydroxytyrosol; and
c. An amount of +/-30% of from 40 mg to 120 mg of curcumin.

As used herein, the term "medicinal food" or "medical food" explicitly refers to a category of substances intended for the clinical dietary management of a particular condition or disease. Specific criteria necessary to receive this FDA designation include that the product must be:
- A specifically formulated food for oral or enteral ingestion;
- For the clinical dietary management of a specific medical disorder, disease or abnormal condition for which there are distinctive nutritional requirements;
- Made with Generally Recognized As Safe (GRAS) ingredients;
- In compliance with FDA regulations that pertain to labeling, product claims and manufacturing.

As a therapeutic category, medical food is distinct from both drugs and supplements. Labels must include the phrase, "to be used under medical supervision," as medical foods are produced under rigid manufacturing practices and maintain high labeling standards. As used herein, the term "dietary supplement composition" explicitly refers to a product taken by mouth that contains a "dietary ingredient" intended to supplement the diet. The "dietary ingredients" in these products may include: vitamins, minerals, herbs or other botanicals, amino acids, and substances such as enzymes, organ tissues, glandulars, and metabolites. Dietary supplements can also be extracts or concentrates, and may be found in many forms such as tablets, capsules, softgels, gelcaps, liquids, or powders. They can also be in other forms, such as a bar, but if they are, information on their label must not represent the product as a conventional food or a sole item of a meal or diet. Whatever their form may be, DSHEA places dietary supplements in a special category under the general umbrella of "foods," not drugs, and requires that every supplement be labeled a dietary supplement.

As used herein, the term "Hydroxytyrosol" is a phenylethanoid, a type of phenolic phytochemical with antioxidant properties in vitro. In nature, hydroxytyrosol is found in olive leaf and olive oil, in the form of its elenolic acid ester oleuropein and, especially after degradation, in its plain form. Its chemical structure is as follows:

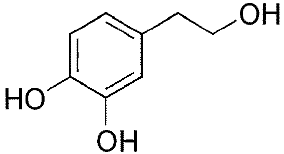

Oleuropein, along with oleocanthal, are responsible for the bitter taste of extra virgin olive oil. Hydroxytyrosol itself in pure form is a colorless, odorless liquid. The olives, leaves and olive pulp contain large amounts of hydroxytyrosol (compared to olive oil), most of which can be recovered to produce hydroxytyrosol extracts.

Hydroxytyrosol is also a metabolite of the neurotransmitter dopamine.

As used herein the term "hydroxytyrosol derivatives or analogues" is understood as esters. It is also possible to use a mixture of hydroxytyrosol and hydroxytyrosol derivatives.

Derivatives or analogues may be e.g. esters known to the person skilled in the art. Preferred esters of hydroxytyrosol are e.g. acetates or gucuronide conjugates, as well as oleuropein being the most preferred one.

As used herein, the term "omega-3 polyunsaturated fatty acid(s)" refers to a family of unsaturated fatty carboxylic acids that have in common a carbon-carbon bond in the n-3 position (i.e., the third bond from the methyl end of the molecule). Typically, they contain from about 16 to about 24 carbon atoms and from three to six carbon-carbon double bonds. Omega-3 polyunsaturated fatty acids can be found in nature, and these natural omega-3 polyunsaturated fatty acids frequently have all of their carbon-carbon double bonds in the cis-configuration. Examples of omega-3 polyunsaturated fatty acids include, but are not limited to, 7,10,13-hexadecatrienoic acid (sometimes abbreviated as 16:3 (n-3)); 9,12,15-octadecatetrienoic acid (α-linolenic acid (ALA), 18:3 (n-3)); 6,9,12,15-octadecatetraenoic acid (stearidonic acid (STD), 18:4 (n-3)); 11,14,17-eicosatrienoic acid (eicosatrienoic acid (ETE), 20:3 (n-3)); 8,11,14,17-eicosatetraenoic acid (eicosatetraenoic acid (ETA), 20:4 (n-3)); 5,8,11,14,17-eicosapentaenoic acid (eicosapentaenoic acid (EPA), (20:5 (n-3)); 7,10,13,16,19-docosapentaenoic acid (docosapentaenoic acid (DPA), 22:5 (n-3)); 4,7,10,13,16,19-docosahexaenoic acid (docosahexaenoic acid (DHA), 22:6 (n-3)); 9,12,15,18,21-tetracosapentaenoic acid (tetracosapentaenoic acid, 24:5 (n-3)); and 6,9,12,15,18,21-tetracosahexaenoic acid (tetracosahexaenoic acid, 24:6 (n-3)).

Eicosapentaenoic acid (EPA) and docosahexaenoic acid (DHA) are found in nature in fish oils and other natural sources, and have been used in a variety of dietary/therapeutic compositions. EPA and DHA are preferred omega-3 polyunsaturated fatty acids in the present invention. The terms "EPA" and "DHA" are used herein indistinctively in two contexts. First they are used in the context of an omega-3 polyunsaturated fatty acid, "EPA" and "DHA" referring to the free acid form of the omega-3 polyunsaturated fatty acid. Secondly they are used in the context of omega-3 polyunsaturated fatty acid derivatives, "EPA" and "DHA" referring to the fact that the derivative contains an eicosapentaenoic acid moiety or docosahexaenoic acid moiety which is present as, for example, an ester, glyceride or phospholipid.

As used herein, the term "curcumin" is also known as diferuloylmethane or (E,E)-l,7-bis (4-hydroxy-3-methoxyphenyl)-l,6-heptadiene-3,5,-dione and has the chemical structure depicted below:

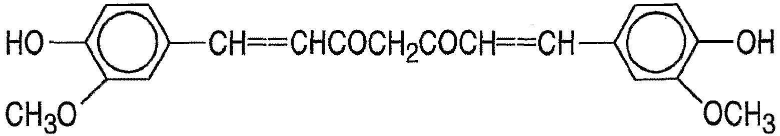

Curcumin may be derived from a natural source, the perennial herb Curcuma longa L., which is a member of the Zingiberaceae family. The spice turmeric is extracted from the rhizomes of Curcuma longa L. and has long been associated with traditional-medicine treatments used in Hindu and Chinese medicine. Turmeric was administered orally or topically in these traditional treatment methods.

Curcumin is soluble in ethanol, alkalis, ketones, acetic acid and chloroform. It is insoluble in water. Curcumin is therefore lipophilic, and generally readily associates with lipids, e.g. many of those used in the colloidal drug-delivery systems of the present invention. In certain embodiments, curcumin can also be formulated as a metal chelate.

As used herein, curcumin analogues are those compounds which due to their structural similarity to curcumin, exhibit anti-proliferative or pro-apoptotic effects on cancer cells similar to that of curcumin. Curcumin analogues which may have anti-cancer effects similar to curcumin include Ar-tumerone, methylcurcumin, demethoxy curcumin, bisdemethoxycurcumin, sodium curcuminate, dibenzoylmethane, acetylcurcumin, feruloyl methane, tetrahydrocurcumin, 1 ,7-bis(4-hydroxy-3 -methoxyphenyl)- 1 ,6-heptadiene-3 ,5-dione (curcuminl ), 1 ,7-bis(piperonyl)- l,6-heptadiene-3,5-dione (piperonyl curcumin) 1,7-bis(2-hydroxy naphthyl)-l,6-heptadiene-2,5- dione (2-hydroxyl naphthyl curcumin), l,l-bis(phenyl)-l,3,8,10 undecatetraene-5,7-dione (cinnamyl curcumin) and the like (Araujo and Leon, 2001; Lin et al, 2001; John et al., 2002; see also Ishida et al, 2002). Curcumin analogues may also include isomers of curcumin, such as the (Z,E) and (Z,Z) isomers of curcumin. In a related embodiment, curcumin metabolites which have anti-cancer effects similar to curcumin can also be used in the present invention. Known curcumin metabolites include glucoronides of tetrahydrocurcumin and hexahydrocurcumin, and dihydroferulic acid. In certain embodiments, curcumin analogues or metabolites can be formulated as metal chelates, especially copper chelates. Other appropriate derivatives of curcumin, curcumin analogues and curcumin metabolites appropriate for use in the present invention will be apparent to one of skill in the art.

It is important to note that although curcumin has shown efficacy against numerous human ailments, poor bioavailability due to poor absorption, rapid metabolism and rapid systemic elimination have been shown to limit its therapeutic efficacy. Because of this reason, numerous efforts have been made to improve curcumin's bioavailability by altering these features. The use of adjuvants that can block the metabolic pathway of curcumin is the most common strategy for increasing the bioavailability of curcumin. In this regard, the effect of combining piperine, a known inhibitor of hepatic and intestinal glucuronidation, with curcumin increased the bioavailability of curcumin by 2.000%. Other promising approaches to increase the bioavailability of curcumin in human include the use of nanoparticles, liposomes, phospholipid complexes and structural analogues.

However, unexpectedly the results provided herein are provided by using a non-formulated mixture of curcumin to manufacture the composition of the invention, namely curcumin powder at least 90%, preferably at least 95% pure which has not been previously formulated to increase its bioavailability by using, for example, curcumin-phospholipid complexes and/or structural analogues. The results are unexpected since, in addition to the fact that the curcumin component has not been previously formulated to increase its bioavailability, none of the other components used to manufacture the final composition used to perform the clinical trial detailed herein, such as hydroxytyrosol, EPA/DHA, Gelatin 98, mono and diglycerides of fatty acids (E471), soybean oil, iron Oxide (E172) or Titanium dioxide (E171), would, a priori, increase the bioavailability of the curcumin component. In addition, soybean lecithin is only known to increase the bioavailability of curcumin if it forms curcumin-phospholipid complexes and the ratio of phospholipids to curcumin is in the range from 10 to 1 w/w.

Therefore, the curcumin component use to manufacture the composition of the present invention is preferably in the form of a curcumin powder at least 90%, preferably at least 95% pure that has not been previously formulated to increase the bioavailability of curcumin in a human subject (such component will be referred to from hereinafter as "a non-formulated curcumin mixture"). More preferably, the final composition of the invention does not contain adjuvants that are known to block the metabolic pathway of curcumin such as piperine, a known inhibitor of hepatic and intestinal glucuronidation, or curcumin-phospholipid complexes wherein the w/w ratio of phospholipids with respect to curcumin is greater than 1, preferably wherein said composition does not contain curcumin-phospholipid complexes in a ratio of phospholipids to curcumin in the range from 10 to 1 w/w, more preferably in the range from 10 to 2 w/w.

More preferably, the final composition does not contain curcumin nanoparticles, liposomes or curcumin structural analogues that increase the bioavailability of curcumin in a human subject.

Consequently, a first aspect of the invention refers to a composition, preferably in the form of a medical food, dietary supplement composition or of a pharmaceutical composition, comprising curcumin, preferably in the form of a non-formulated curcumin mixture, Omega-3 polyunsaturated fatty acids (PUFAs) EPA and DHA, in a weight ratio of EPA:DHA of from 1.2 to 1.8, and hydroxytyrosol in the amounts as defined in the claims. The daily dosages amounts of each of the active ingredients of the composition of the invention are:
1. An amount of +/-30% of from 243.8 mg to 731.4 mg of Omega-3 polyunsaturated fatty acids (PUFAs) EPA and DHA, in a weight ratio of EPA:DHA of from 1.2 to 1.8;
2. An amount of +/-30% of from 12.5 mg to 37.5 mg of hydroxytyrosol; and
3. An amount of +/-30% of from 40 mg to 120 mg of a non-formulated curcumin mixture.

Preferably, daily dosage amounts of the active ingredients of the invention are +/-30% of 731.4 mg/day of Omega-3 polyunsaturated fatty acids (PUFAs) EPA and DHA, +/-30% of 37.5 mg/day of hydroxytyrosol and +/-30% of 120 mg/day of curcumin, preferably a non-formulated curcumin mixture.

Preferably, suitable dosage amounts of the active ingredients of the invention are +/-20% of 731.4 mg/day of Omega-3 polyunsaturated fatty acids (PUFAs) EPA and DHA, +/-20% of 37.5 mg/day of hydroxytyrosol and +/-20% of 120 mg/day of curcumin, preferably a non-formulated curcumin mixture.

More preferably, suitable dosage amounts of the active ingredients of the invention are +/-10% of 731.4 mg/day of Omega-3 polyunsaturated fatty acids (PUFAs) EPA and DHA,.+/-10% of 37.5 mg/day of hydroxytyrosol and +/-10% of 120 mg/day of curcumin, preferably a non-formulated curcumin mixture.

Still, more preferably, suitable dosage amounts of the active ingredients of the invention are +/-5% of 731.4 mg/day of Omega-3 polyunsaturated fatty acids (PUFAs) EPA and DHA, +/-5% of 37.5 mg/day of hydroxytyrosol and +/-5% of 120 mg/day of curcumin, preferably a non-formulated curcumin mixture.

In a preferred embodiment of the invention, the composition comprises:
a. An amount of +/-30% of 731.4 mg of Omega-3 polyunsaturated fatty acids (PUFAs) EPA and DHA, in a weight ratio of EPA:DHA of from 1.2 to 1.8, wherein this amount can be preferably administered in 2 or three daily dosages each comprising +/-30% of 243.8 mg (for three daily dosages) or +/-30% of 365.7 mg (for two daily dosages) of Omega-3 polyunsaturated fatty acids (PUFAs) EPA and DHA ;
b. An amount of +/-30% of 37.5 mg of hydroxytyrosol wherein this amount can be preferably administered in 2 or three daily dosages each comprising +/-30% of 12.5 mg (for three daily dosages) or +/-30% of 18.75 mg (for two daily dosages) of hydroxytyrosol; and
c. An amount of +/-30% of 120 mg of curcumin, wherein this amount can be preferably administered in 2 or three daily dosages each comprising +/-30% of 40 mg (for three daily dosages) or +/-30% of 60 mg (for two daily dosages) of curcumin, preferably a non-formulated curcumin mixture.

In a more preferred embodiment of the invention, the composition comprises:
a. An amount of +/-20% of 731.4 mg of Omega-3 polyunsaturated fatty acids (PUFAs) EPA and DHA, wherein this amount can be preferably administered in 2 or three daily dosages;
b. An amount of +/-20% of 37.5 mg of hydroxytyrosol, wherein this amount can be preferably administered in 2 or three daily dosages; and
c. An amount of +/-20% of 120 mg of curcumin, preferably a non-formulated curcumin mixture, wherein this amount can be preferably administered in 2 or three daily dosages.

In a more preferred embodiment of the invention, the composition comprises:
a. An amount of +/-10% of 731.4 mg of Omega-3 polyunsaturated fatty acids (PUFAs) EPA and DHA, wherein this amount can be preferably administered in 2 or three daily dosages;
b. An amount of +/-10% of 37.5 mg of hydroxytyrosol, wherein this amount can be preferably administered in 2 or three daily dosages; and
c. An amount of +/-10% of 120 mg of curcumin, preferably a non-formulated curcumin mixture, wherein this amount can be preferably administered in 2 or three daily dosages.

In a more preferred embodiment of the invention, the composition comprises:
a. An amount of +/-5% of 731.4 mg of Omega-3 polyunsaturated fatty acids (PUFAs) EPA and DHA, wherein this amount can be preferably administered in 2 or three daily dosages;
b. An amount of +/-5% of 37.5 mg of hydroxytyrosol, wherein this amount can be preferably administered in 2 or three daily dosages; and
c. An amount of +/-5% of 120 mg of curcumin, preferably a non-formulated curcumin mixture, wherein this amount can be preferably administered in 2 or three daily dosages.

In a more preferred embodiment of the invention, the composition comprises:
a. An amount of about 731.4 mg of Omega-3 polyunsaturated fatty acids (PUFAs) EPA and DHA, wherein this amount can be preferably administered in 2 or three daily dosages;
b. An amount of about 37.5 mg of hydroxytyrosol, wherein this amount can be preferably administered in 2 or three daily dosages; and
c. An amount of about 120 mg of curcumin, preferably a non-formulated curcumin mixture, wherein this amount can be preferably administered in 2 or three daily dosages.

In the context of the present invention, the term "about" explicitly refers to percentages of +/-1% of the indicated amount.

In a still more preferred embodiment of the invention, the composition is a capsule comprising the following active ingredients in about the proportions and quantities specified in the table below:

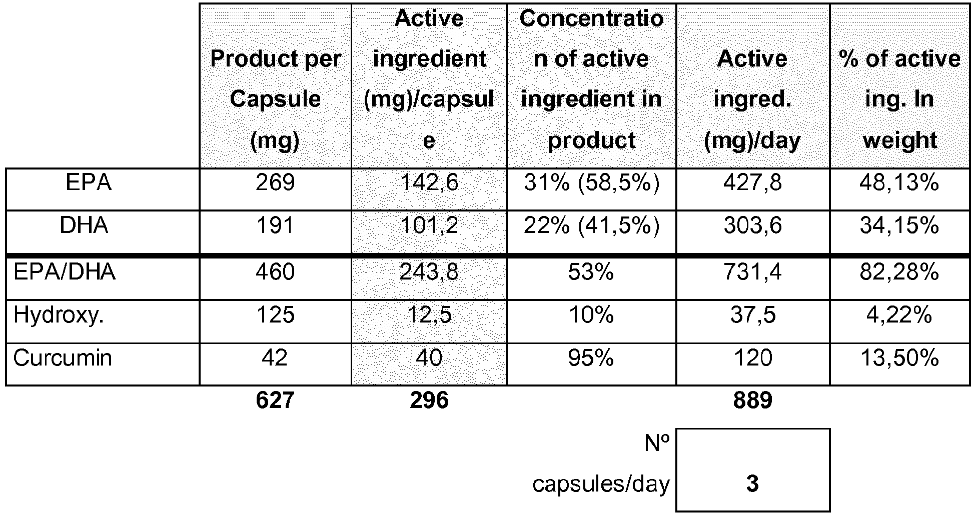

In a still more preferred embodiment of the invention, the composition is a capsule comprising the following active ingredients and excipients:

| | **mg/capsule** | **g/100g** |
|---|---|---|
| Fish oil (Triglycerid form) 310 mg/g EPA | | |
| and 220 mg/g DHA (ONC) | 460 | 55 |
| Hytolive 10% powder | 125 | 15 |
| Gelatin 98. | 9 | 12 |
| Mono and diglycerides of fatty acids (E471) | 50.0 | 6.0 |
| Curcumin Powder 95% | 42.0 | 5.0 |
| Soybean oil, refined | 28.0 | 3.3 |
| Water | 16.8 | 2.0 |
| Soybean lecithin solubilized in Soya | | |
| oil,enriched with phosphatidylcholine | 15.0 | 1.8 |
| Iron Oxide (E172) | 1.77 | 0.21 |
| Titanium dioxide (E171) | 0.590 | 0.070 |

In a preferred embodiment of the first aspect of the invention or of any of its preferred embodiments, the composition is a pharmaceutical, medical food or dietary supplement composition.

### - Doses and administration of the pharmaceutical, medical food or dietary supplement of the invention

The composition of the invention comprises the components in biologically and pharmaceutically active amounts, that is amounts sufficient to achieve the desired health promoting effect, namely the reduction in CRP levels in plasma. As will be readily understood by a physician, the amounts will vary depending on the individual and his or her health status as well as on other factors such as weight, age, nutrition, stress, environmental factors, etc... Variations of up to +/- 30% of the daily dosages of each of the active ingredients indicated herein are understood to achieve the desired health promoting effect, namely the reduction in CRP levels in plasma.

Thus, examples of suitable amounts for a daily administration include, about +/-30% of 731.4 mg/day of Omega-3 polyunsaturated fatty acids (PUFAs) EPA and DHA, +/-30% of 37.5 mg/day of hydroxytyrosol and +/-30% of 120 mg/day of curcumin. Merely as an example, these daily amounts can be easily provided by administering the following composition three times per day:

| | **mg/capsule** | **g/100g** |
|---|---|---|
| Fish oil (Triglycerid form) 310 mg/g EPA and 220 mg/g DHA (ONC) | 460 | 55 |
| Hytolive 10% powder | 125 | 15 |
| Gelatin 98. | 9 | 12 |
| Mono and diglycerides of fatty acids (E471) | 50.0 | 6.0 |
| Curcumin Powder 95% | 42.0 | 5.0 |
| Soybean oil, refined | 28.0 | 3.3 |
| Water | 16.8 | 2.0 |
| Soybean lecithin solubilized in Soya | | |
| oil,enriched with phosphatidylcholine | 15.0 | 1.8 |
| Iron Oxide (E172) | 1.77 | 0.21 |
| Titanium dioxide (E171) | 0.590 | 0.070 |

Other suitable compositions for administering the above state daily amounts will be apparent to the skilled artisan in the art.

All the components are administered orally, preferably in connection with meals as a dietary supplementation composition. They may be administered separately, or in variable combinations. They may be purchased e.g. in powder form separately, or as ready-made powders containing all ingredients such as the capsules used through-out the examples of the present invention. Such a powder mixture may be pre-packed and used as such or as a supplement to conventional food items e.g. in a dairy product such as yoghurt or ice cream. Of course the pharmaceutical composition may also be processed into granulates, capsules or tablets, which may comprise pharmaceutically acceptable carriers. Conveniently it is in the form of capsules. The dietary composition of the present invention can be administered either simultaneously with the other ingredients or separately at different times.

Thus, a second aspect of the invention refers to a composition comprising Omega-3 polyunsaturated fatty acids (PUFAs) EPA and DHA, in a weight ratio of EPA:DHA of from 1.2 to 1.8, hydroxytyrosol and curcumin, preferably a non-formulated curcumin mixture, for use in a method of reducing CRP in plasma, wherein this composition is administered orally, in the amounts as defined in claim 1.

A preferred embodiment of the second aspect of the invention refers to a composition comprising Omega-3 polyunsaturated fatty acids (PUFAs) EPA and DHA, in a weight ratio of EPA:DHA of from 1.2 to 1.8, hydroxytyrosol and curcumin, preferably a non-formulated curcumin mixture, for use in a method of reducing CRP in plasma, wherein said composition is administered in one or more daily dosages so that the daily amount of each of the three components is +/-30% of 731.4 mg of Omega-3 polyunsaturated fatty acids (PUFAs) EPA and DHA, +/-30% of 37.5 mg of hydroxytyrosol and +/-30% of 120 mg of curcumin, preferably a non-formulated curcumin mixture, and wherein said composition is administered orally.

Another preferred embodiment of the second aspect of the invention refers to a composition comprising Omega-3 polyunsaturated fatty acids (PUFAs) EPA and DHA, in a weight ratio of EPA:DHA of from 1.2 to 1.8, hydroxytyrosol and curcumin, preferably a non-formulated curcumin mixture, for use in a method of reducing CRP in plasma, wherein said composition is administered in one or more daily dosages so that the total daily amount of each of the three active ingredients is +/-20% of 731.4 mg of Omega-3 polyunsaturated fatty acids (PUFAs) EPA and DHA, +/-20% of 37.5 mg of hydroxytyrosol and +/-20% of 120 mg of curcumin, preferably a non-formulated curcumin mixture, and wherein said composition is administered orally.

Another preferred embodiment of the second aspect of the invention refers to a composition comprising Omega-3 polyunsaturated fatty acids (PUFAs) EPA and DHA, in a weight ratio of EPA:DHA of from 1.2 to 1.8, hydroxytyrosol and curcumin, preferably a non-formulated curcumin mixture, for use in a method of reducing CRP in plasma, wherein said composition is administered in one or more daily dosages so that the total daily amount of each of the three active ingredients is +/-10% of 731.4 mg of Omega-3 polyunsaturated fatty acids (PUFAs) EPA and DHA, +/-10% of 37.5 mg of hydroxytyrosol and +/-10% of 120 mg of curcumin, preferably a non-formulated curcumin mixture, and wherein said composition is administered orally.

Another preferred embodiment of the second aspect of the invention refers to a composition comprising Omega-3 polyunsaturated fatty acids (PUFAs) EPA and DHA, in a weight ratio of EPA:DHA of from 1.2 to 1.8, hydroxytyrosol and curcumin, preferably a non-formulated curcumin mixture, for use in a method of reducing CRP in plasma, wherein said composition is administered in one or more daily dosages so that the total daily amount of each of the three active ingredients is +/-5% of 731.4 mg of Omega-3 polyunsaturated fatty acids (PUFAs) EPA and DHA, +/-5% of 37.5 mg of hydroxytyrosol and +/-5% of 120 mg of curcumin, preferably a non-formulated curcumin mixture, and wherein said composition is administered orally.

Another preferred embodiment of the second aspect of the invention refers to a composition comprising Omega-3 polyunsaturated fatty acids (PUFAs) EPA and DHA, in a weight ratio of EPA:DHA of from 1.2 to 1.8, hydroxytyrosol and curcumin, preferably a non-formulated curcumin mixture, for use in a method of reducing CRP in plasma, wherein said composition is administered in one or more daily dosages so that the total daily amount of each of the three active ingredients is about 731.4 mg of Omega-3 polyunsaturated fatty acids (PUFAs) EPA and DHA, about 37.5 mg of hydroxytyrosol and about 120 mg of curcumin, preferably a non-formulated curcumin mixture, and wherein said composition is administered orally.

### - Manufacturing process

The skilled person will certainly know how to manufacture the compositions described in present invention. In any case and merely for illustrative purposes one non-limited manner of producing an encapsulated composition of the invention is generally described as follows:

### General manufacturing process

1. Preparing the capsule mass and the filling preparation by using any method known to the skilled person;
2. Encapsulating the filling preparation with the capsule mass;
3. Drying the mixture;
4. Sorting and packaging.

Possible additives and shell components useful to produce a capsule of the present invention are illustrated below:

### Additives:

- Palm oil (filling agent);
- Beeswax (thickening agent);
- Mono-diglicerides from fatty acids (thickening agent);
- Soya lecithin (emulsifier); and
- Coloidal silica (thickening agent);

### Shell components of the capsule:

- Gelatin (gelling agent);
- Glycerine (humectant);
- Iron oxide (pigment);
- Titanium dioxide (pigment);
- Carmine E120 (pigment)

### - Further specific Embodiments of the invention

The invention relates to the dietary treatment and prophylaxis of cancer. In particular the invention relates to several metabolic agents acting in synergy as a signal system regulating the genome. These spontaneous complexes of Omega-3 polyunsaturated fatty acids (PUFAs) EPA and DHA, hydroxytyrosol and curcumin and/or curcumin analogues have a therapeutic effect. They have been successfully used for the treatment and prophylaxis of cancer, in particular of breast cancer. Promising results have been achieved in increasing the overall survival rate of breast cancer patients.

Thus, a third aspect of the invention refers to a composition comprising Omega-3 polyunsaturated fatty acids (PUFAs) EPA and DHA, weight ratio of EPA:DHA of from 1.2 to 1.8, hydroxytyrosol and/or hydroxytyrosol analogues and curcumin, preferably a non-formulated curcumin mixture, for use in a method of increasing the overall survival rate of breast cancer patients diagnosed with said disease, wherein this composition is administered orally, in the amounts as defined in claim 1.

A preferred embodiment of the third aspect of the invention refers to a composition comprising Omega-3 polyunsaturated fatty acids (PUFAs) EPA and DHA, hydroxytyrosol and curcumin, preferably a non-formulated curcumin mixture, for use in a method of increasing the overall survival rate of breast cancer patients diagnosed with said disease, wherein this composition is administered orally and wherein said composition is administered in one or more daily dosages so that the daily amount of each of the three components is +/-30% of 731.4 mg of Omega-3 polyunsaturated fatty acids (PUFAs) EPA and DHA, +/-30% of 37.5 mg of hydroxytyrosol and +/-30% of 120 mg of curcumin, preferably a non-formulated curcumin mixture, and wherein said composition is administered orally.

Another preferred embodiment of the third aspect of the invention refers to a composition comprising Omega-3 polyunsaturated fatty acids (PUFAs) EPA and DHA, hydroxytyrosol and curcumin, preferably a non-formulated curcumin mixture, for use in a method of increasing the overall survival rate of breast cancer patients diagnosed with said disease, wherein this composition is administered orally and wherein said composition is administered in one or more daily dosages so that the daily amount of each of the three components is +/-20% of 731.4 mg of Omega-3 polyunsaturated fatty acids (PUFAs) EPA and DHA, +/-20% of 37.5 mg of hydroxytyrosol and +/-20% of 120 mg of curcumin, preferably a non-formulated curcumin mixture, and wherein said composition is administered orally.

Another preferred embodiment of the third aspect of the invention refers to a composition comprising Omega-3 polyunsaturated fatty acids (PUFAs) EPA and DHA, hydroxytyrosol and curcumin, preferably a non-formulated curcumin mixture, for use in a method of increasing the overall survival rate of breast cancer patients diagnosed with said disease, wherein this composition is administered orally and wherein said composition is administered in one or more daily dosages so that the total daily amount of each of the three active ingredients is +/-10% of 731.4 mg of Omega-3 polyunsaturated fatty acids (PUFAs) EPA and DHA, +/-10% of 37.5 mg of hydroxytyrosol and +/-10% of 120 mg of curcumin, preferably a non-formulated curcumin mixture, and wherein said composition is administered orally.

Another preferred embodiment of the third aspect of the invention refers to a composition comprising Omega-3 polyunsaturated fatty acids (PUFAs) EPA and DHA, hydroxytyrosol and curcumin, preferably a non-formulated curcumin mixture, for use in a method of increasing the overall survival rate of breast cancer patients diagnosed with said disease, wherein this composition is administered orally and wherein said composition is administered in one or more daily dosages so that the total daily amount of each of the three active ingredients is +/-5% of 731.4 mg of Omega-3 polyunsaturated fatty acids (PUFAs) EPA and DHA, +/-5% of 37.5 mg of hydroxytyrosol and +/-5% of 120 mg of curcumin, preferably a non-formulated curcumin mixture, and wherein said composition is administered orally.

Another preferred embodiment of the third aspect of the invention refers to a composition comprising Omega-3 polyunsaturated fatty acids (PUFAs) EPA and DHA, hydroxytyrosol and curcumin, preferably a non-formulated curcumin mixture, for use in a method of increasing the overall survival rate of breast cancer patients diagnosed with said disease, wherein this composition is administered orally and wherein said composition is administered in one or more daily dosages so that the total daily amount of each of the three active ingredients is about 731.4 mg of Omega-3 polyunsaturated fatty acids (PUFAs) EPA and DHA, about 37.5 mg of hydroxytyrosol and about 120 mg of curcumin, preferably a non-formulated curcumin mixture, and wherein said composition is administered orally.

Methods of treating or preventing cancer with the pharmaceutical, medical food or dietary supplement pharmaceutical composition are disclosed comprising administering an effective amount of the pharmaceutical, medical food or dietary supplement to a person in need thereof. Prevention as used herein refers to the clinical outcome, which is "overall survival" (OS). "Overall survival" denotes the chances of a cancer patient, in particular of a breast cancer patient, of staying alive for a group of individuals suffering from a cancer. The decisive question is whether the individual is dead or alive at a given time point. The inventors have shown that reducing the CRP levels in plasma one or more octiles is indicative of overall survival.

The following examples have been inserted herein for illustration purposes only and thus do not limit the present invention.

### EXAMPLES

### Example 1. Design of the clinical study

- Pilot clinical trial to assess changes in biomarkers of cancer related to inflammation in women with stage 0-IIIA breast cancer and without evidence of disease were given the dietary complement composition of the invention (investigational product).

### 1.1. Description of the investigational product

| | **Experimental** |
|---|---|
| COMPOSITION (per capsule): | • 460 mg of fish oil (EPA and DHA) |
| | • 125 mg Hytolive^{™} powder (12.5 mg of hydroxytyrosol) |
| | • 42 mg extract of curcumin (40 mg curcuminoids) in a non-formulated form. |
| DOSE: | Two capsules in the morning, and one capsule at night, every day, by oral administration taken with a glass of water for one month. |
| ROUTE OF ADMINISTRATION: | Oral |
| FORM: | Capsule |
| MANUFACTURER: | Capsugel |

In particular, the patients were administered three capsules per day of the following pharmaceutical, medical food or dietary supplement composition per capsule:

| | **mg/capsule** | **g/100g** |
|---|---|---|
| Fish oil (Triglycerid form) 310 mg/g EPA and 220 mg/g DHA (ONC) | 460 | 55 |
| Hytolive 10% powder | 125 | 15 |
| Gelatin 98. | 9 | 12 |
| Mono and diglycerides of fatty acids (E471) | 50.0 | 6.0 |
| Curcumin Powder 95% | 42.0 | 5.0 |
| Soybean oil, refined | 28.0 | 3.3 |
| Water | 16.8 | 2.0 |
| Soybean lecithin solubilized in Soya oil,enriched with phosphatidylcholine | 15.0 | 1.8 |
| Iron Oxide (E172) | 1.77 | 0.21 |
| Titanium dioxide (E171) | 0.590 | 0.070 |

### 1.2. Experimental phase

- Single-arm, single-cohort pilot trial. No control group.
- During the selection period, two blood samples were extracted per patient (5 +/- 2 days part), with 10 mL of peripheral blood drawn in each extraction. 5 mL were used for routine analyses. Serum was extracted from the other 5 mL and stored at -80°C for subsequent analysis.
- The beginning of treatment started no later than 28 days after the date of the first extraction of the selection period. Therefore, the treatment with the investigational product began on day 0 of the trial. The patients signed their informed consent to be included in the trial and received the medication for one month. The capsules of the investigational product were administered orally as follows: 3 capsules a day for 1 month, 2 in the morning and 1 at night for one month (30 days).
- On day 14 of treatment each patient was evaluated, in this sense a clinical history was made and they were questioned on adverse events or toxicity related to the taking of the investigational product. Each patient provided their BPI scale.
- At the end-of-treatment visit, on day 30, a Clinical History was again made, and each patient provided their patient diary along with the BPI Scale completed. At approximately that time, two further extractions were performed, on day 30 and day 33 (+/- 2 days), with 10 mL of blood taken per extraction that were processed as described above.
- Finally, on day 60 from day 0 of the trial, the patients were asked about their general condition and whether there has been any adverse event, related or not to the medication. At that date, one further extraction was performed per patient, with 10 mL of blood taken that was processed as described above.
- All the serum samples were frozen and stored at -80°C in the laboratory. Once the trial has been completed the samples were sent to the IDMEA Food Laboratory for the subsequent analysis of inflammation biomarkers.
- The following determinations were performed in each serum sample: CRP, IL-6, SAA, IFN_{gamma}, and TNF-alpha, IL-10 and TGF_{beta} and IGF-1.
- The effect on cholesterol and triglycerides was also measured.
- The BPI pain scale was provided to each patient at their visits on day 0, day 14 and day 30.

### 1.3. Inclusion criteria

1. Women with histologically confirmed AJCC Stage 0-IIIA breast cancer which has been completely surgically resected.
2. No evidence of disease as determined by their physician.
3. ER+ and/or PR+ tumour.
4. Receiving an aromatase inhibitor (letrozole, anastrazole, exemestane) or tamoxifen at a stable dose for at least 3 months at trial entry.
5. Post-menopausal women, defined as: (1) above 50 years of age who have not menstruated during the preceding 12 months or who have follicle-stimulating hormone levels (FSH) > 40 IU/L, (2) those under 50 years of age who have FSH hormone levels >40 IU/L, or (3) those who have undergone a bilateral oophorectomy.
6. CRP ≥3.9 mg/L measured as the mean of two consecutive weekly tests.
7. Aged 18 years or older.
8. ECOG performance status 0-1. These scales and criteria are used by doctors and researchers to assess how a patient's disease is progressing, assess how the disease affects the daily living abilities of the patient, and determine appropriate treatment and prognosis. They are included here for health care professionals to access.
9. A time interval between 2 and 5 years from their initial surgery for breast cancer.
10. Life expectancy of at least 6 months
11. At least 6 months since last chemotherapy
12. Laboratory tests performed within 14 days of enrolment in the trial:
   a. Granulocytes ≥ 1,500/µL;
   b. Platelets ≥ 100,000/µL;
   c. Haemoglobin ≥ 12.0 g/dL;
   d. Total bilirubin equal to or below upper limit of normal (ULN);
   e. AST and ALT equal to or below ULN;
   f. Alkaline phosphatase equal to or below ULN;
   g. Serum creatinine equal to or below ULN;
13. Able to provide informed consent to receive the study treatment, to provide biological specimens, self-administration of oral medication unsupervised for a prolonged period of time, and to complete a medication diary.

### 1.4. Exclusion criteria

1. Pregnancy or breastfeeding.
2. Having had a malignancy (other than breast cancer) which required radiotherapy or systemic treatment within the past 5 years.
3. Known cardiac disease (arrhythmias, myocardial infarction, bundle branch block, ischemic heart disease, and uncontrolled hypertension).
4. Known autoimmune disease or inflammatory disorder.
5. Any condition requiring the use of systemic corticosteroids or any other immunosuppressive agents (e.g. cyclosporin, tacrolimus, azathriopine).
6. Women with known immunodeficiency (such as HIV).
7. Patients with infection by septicaemia, infection, acute hepatitis, or other uncontrolled severe medical condition.
8. Routine use of aspirin >81 mg/d or NSAIDs (> 400 mg po 4 times/day of ibuprofen or naproxen > 500mg/d) or any use of celecoxib or similar COX-2 inhibitors;
9. Subjects were asked not to take dietary supplements, olives or olive oil for 1 month prior to study enrolment and during the study.
10. Taking medication containing bisphosphonates.

### 1.5. Selection of the participating subjects

Once the patients that fulfilled all the inclusion criteria and none of the exclusion criteria have been selected they will be asked to sign the informed consent form to be included in the trial.

### 1.6. Diagnostic criteria

### - Pre-treatment procedures

As already stated, two blood samples were drawn from each patient before treatment began (5 +/- 2 days apart). All blood samples were drawn during the morning hours, between 7 am and 10 am under fasting conditions for 8 hours.

The treatment with the investigational product began no later than 28 days after the first blood sample was taken during the selection period.

At each sampling, 10 mL of peripheral blood were drawn and inserted into two (2) 5 mL red-top Vacutainer tubes. One of the tubes was sent to the hospital laboratory for a routine analysis:
- Cell count: red blood cells, haemoglobin, leukocytes and platelets;
- C-Reactive Protein;
- Biochemistry;
- Lipid profile;
- PT; and
- PPT

The other tube was rapidly centrifuged (2 to 3 hours after sampling), to separate the serum from the "*buffy coat*". Once the serum was obtained (approx. 2 mL of the 5 mL of blood), it was distributed in ten aliquots of 200 microliters each in small Eppendorf tubes, suitably labelled, and frozen at -80°C. These samples were used for the subsequent analysis of the following inflammation biomarkers:
- IL-6;
- SAA; - Serum Amyloid -A
- IFN_{gamma;}
- TNF-alpha;
- IL-10;
- TGF_{beta;}
- IGF-1; and
- ox-LDL.

At the **end of the trial** the procedure referred to herein was repeated, with the drawings of two further blood samples (3 +/- 2 days apart) and the serum samples obtained and stored at -80°C.

Finally, all serum aliquots were sent to the Food Laboratory of the IMDEA (Instituto Madrileño de Estudios Avanzados) for the analysis of the inflammation and ox-LDL biomarkers.

### 1.7. Other supplements (Wash-out period)

Patients were asked to stop taking any other food supplement and limit the use of olives or olive oil and all analgesics (except paracetamol) and anti-inflammatory medication for 1 month before the start of the trial, i.e. before the first extraction (washout). Patients were allowed to take paracetamol (650 mg capsules) for severe pain during the trial.

### 1.8. Number of subjects

Thirty-two (32) women with stage 0-IIIA breast cancer have enrolled and finished the clinical trials thus far.

### 1.9. Methodological criteria

Patients followed the investigator's recommendations for taking the investigational product.

### 1.10. Criteria for postponing the administration of the treatment to patients.

- If any of the following criteria arises while the patient is enrolled in the trial, the beginning of the treatment was deemed postponed:
   1) Acute illness at the time of the investigational product cycle initiation. Acute illness is defined as the presence of a moderate or severe illness with or without fever, as well as minor illness such as diarrhoea or mild upper respiratory infection which can affect inflammatory markers.
   2) Fever, defined as an oral or axillary temperature of 38°C or above.
   3) Any other grade 1 or higher toxicities (according to CTCAE (Common Terminology Criteria for Adverse Events, Version 4.0)
- Criteria for resuming treatment after postponement:
   - If treatment administration is postponed, the subject may start at least 1 week after resolution of the clinical symptoms of the acute illness if they have no fever and have no toxicity greater than grade 1.
   - If the treatment with the investigational product is postponed for ≤ 2 days, it may be resumed at the same dose. If the postponement is longer than 2 days, the subject will be withdrawn from the trial and replaced.

### 1.11. Criteria for the permanent suspension of the trial treatment administration

If any of the following criteria becomes applicable during the trial, the patient is required to discontinue the investigational product treatment:
1. Evidence of disease recurrence with the investigator's decision to stop current therapy.
2. Treatment with one of the following:
   - Any other investigational product or non-registered product
   - Anticancer treatments other than the treatments allowed by the protocol, including but not limited to chemotherapeutic or immunomodulatory agents
   - Systemic corticosteroids or any other immunosuppressive agents or use of NSAIDs.
   - Administration of a vaccine.
3. Administration of immunoglobulins during the trial period.
4. Any grade 2 or higher adverse event, according to CTCAE, Version 4.0.
5. Acute illness, defined as the presence of a moderate or severe illness with or without fever as well as minor illness such as diarrhoea or mild upper respiratory infection which can affect inflammatory markers.
6. Fever, defined as an oral or axillary temperature of 38°C or above.
7. Development of an inflammatory condition as determined by the subject's physician.
8. The patient develops other conditions for which, in the investigator's opinion, it is in the patient's best interest to be withdrawn from the treatment. Patients may be eliminated from the ATP population for CRP level analysis if, during the trial, they incur a condition that has the capability of altering their immune response.
9. The patient requests to be withdrawn from treatment.
10. For female patients, pregnancy or the decision to become pregnant. For patients whose treatment is discontinued prematurely during the trial for any reason other than disease progression the Concluding Visit procedures will be carried out at least 30 days following the last administration of THE COMPOSITION.

Patients should receive medication appropriate to their health condition during the whole trial.

At each trial visit/contact, the investigator should question the patient about any medication taken and treatment received by the patient.

All concomitant medication, including changes in chronic medication, including vitamins and/or dietary supplements, are to be recorded in the CRF. This also applies to any medication intended to treat an AE.

### Example 2. EVALUATION OF RESPONSE AND DEVELOPMENT OF THE TRIAL

### 2.1. ENDPOINTS

### Primary endpoint:

- Reduction in the levels of CRP, in comparison with baseline values.

### Secondary variables:

- Reduction in IL-6, SAA, IFN_{gamma} and TNF-alpha. Increase in levels of IL-10 and TGF_{beta}, and reduction in IGF-compared to the baseline analysis.
- Safety and tolerability (GI symptoms)
- Scores of mean pain intensity with stable administration, measured with the BPI scale
- Effect on LDL, HLD, ox-LDL and triglycerides.

### Safety endpoint:

- Adverse events,
- Blood analyses at the beginning and the end of treatment with the investigational product in terms of hepatic and renal profiles.

### 2.2. Results

The results of the present clinical trial in connection to the primary endpoint, namely the reduction in the levels of CRP, in comparison with baseline values, are shown in Tables I and III above.

In addition, the following results have been further obtained by using 44 patients in connection to the reduction in the levels of CRP, in comparison with baseline values.

**Table IV**

| **Statistical Descriptive** | | | | | |
|---|---|---|---|---|---|
| | N | Media | Standard deviation | Mín | Max |
| media1 | 44 | 7,4700 | 4,12659 | 3,92 | 19,82 |
| PR1 | 44 | 7,4681 | 4,12655 | 3,92 | 19,82 |
| A1 | 44 | 7,8039 | 6,00584 | 1,08 | 30,70 |
| A2 | 44 | 7,1323 | 4,46510 | 1,60 | 24,50 |
| media2 | 44 | 5,1852 | 3,21400 | ,67 | 15,26 |
| A5 | 38 | 5,5755 | 4,35905 | ,60 | 20,90 |
| PR2 | 36 | 5,5106 | 3,36140 | 1,00 | 15,26 |
| A3 | 41 | 5,0932 | 3,03724 | ,80 | 13,00 |
| A4 | 39 | 5,5810 | 4,37389 | ,67 | 23,40 |

A1-A5 are the results of five analysis.

Media1 -2 are the means (A1 A2) and (A3 A4) in the data

PR1-2 are the means (A1 A2) y (A3 A4) excluding the missing data.

A Wilcoxon test was performed to determine the significance of the effect. This test is a non-parametric one that contrasts the differences of the means of paired data.

**Table V**

| **Statistics of Contrast ( Wilcoxon)** | | | | | | |
|---|---|---|---|---|---|---|
| | media2 - media 1 | PR -PR1 | A3 - A1 | A4 - A2 | A5-media2 | PR2 - A5 |
| Z Sig. asintót. (bilateral) | -3,085(a) ,002 | -2,326(a) ,020 | -1,996(a) ,046 | -2,805(a) ,005 | -,500(b) ,617 | -,299(b) ,765 |

| | | | | | | |
|---|---|---|---|---|---|---|
| a Based in positive range. b Based in negative range. | | | | | | |

As illustrated in table V above, the treatment decreases both the individual values measured in the tests, as well as the mean of the values.

Conclusions of these further results:
1. There is a statistically significant CRP reduction after the treatment compared with pre- treatment. (p=0.002).
2. There is also a statistically significant reduction in the CRP, comparing individual values, instead of means. That is there is a CRP reduction when comparing analysis 3 to analysis 1; and analysis 4 to analysis 2.
3. The effect might be stable over the time (d+60), as the CRP is at the same level as in post-treatment.

Lastly, results directed to the secondary variable: scores of mean pain intensity with stable administration measured with the BPI scale, have been obtained in 30 out of the 32 woman with stage 0-IIIA breast cancer participating in this clinical trial. Particularly, a significant pain relief has been obtained as shown below by using the dietary complement composition of the invention (investigational product).

**Table VI**

| **Presence of pain** | | | | |
|---|---|---|---|---|
| | | Follow-up | | |
| | | No | Yes | Total |
| Pre-treatment | No | 7 | 2 | 9 |
| | Yes | 7 | 14 | 21 |
| Total | | 14 | 16 | 30 |

As shown in figure 1, there is a statistical significant decrease of the patients that reported pain before the treatment after treatment with the investigational product.

For the measurement of the severity of the pain we used the severity Index in approved and validated tests for the measurement of cancer related pain. The results from these validated tests are shown in the table below.

**Table VII**

| **Severity index** | | | | |
|---|---|---|---|---|
| | Pre-reatment | End of treatment | Shift Pre-treatment-End of treatment | p-value* |
| Mean | 3,15 | 2,08 | -1,07 | 0.049 |
| Median | 3,38 | 1,75 | -,50 | |
| Std. Dev. | 2,20 | 2,12 | 2,27 | |
| Minimum | ,00 | ,00 | -6,50 | |
| Maximum | 8,50 | 7,50 | 2,50 | |
| Percentil 25 | 1,75 | ,00 | -1,75 | |
| Percentil 75 | 4,25 | 3,50 | ,00 | |
| N | 22 | 22 | 22 | |

| | | | | |
|---|---|---|---|---|
| *Wilcoxon test. | | | | |

As shown in figure 2, additionally to the decrease of the number of patients reporting pain, the results of the severity index test indicate that there is a statistically significant decrease in the severity of the pain reported by the patients after the treatment.

## Claims

1. A composition wherein said composition comprises each of elements a) to c) in the following amounts:
a. An amount of +/-30% of from 243.8 mg to 731.4 mg of Omega-3 polyunsaturated fatty acids (PUFAs) EPA and DHA, in a weight ratio of EPA:DHA of from 1.2 to 1.8;
b. An amount of +/-30% of from 12.5 mg to 37.5 mg of hydroxytyrosol; and
c. An amount of +/-30% of from 40 mg to 120 mg of curcumin.

2. The composition of claim 1, wherein said composition comprises each of elements a) to c) in the following amounts:
a. An amount of +/-30% of 731.4 mg of Omega-3 polyunsaturated fatty acids (PUFAs) EPA and DHA, in a weight ratio of EPA:DHA of from 1.2 to 1.8;
b. An amount of +/-30% of 37.5 mg of hydroxytyrosol; and
c. An amount of +/-30% of 120 mg of curcumin.

3. The composition of claim 1, wherein said composition comprises each of elements a) to c) in the following amounts:
a. An amount of +/-30% of 243.8 mg of Omega-3 polyunsaturated fatty acids (PUFAs) EPA and DHA, in a weight ratio of EPA:DHA of from 1.2 to 1.8;
b. An amount of +/-30% of 12.5 mg of hydroxytyrosol; and
c. An amount of +/-30% of 40 mg of curcumin.

4. The composition of claim 1, wherein said composition comprises each of elements a) to c) in the following amounts:
a. An amount of +/-30% of 365.7 mg of Omega-3 polyunsaturated fatty acids (PUFAs) EPA and DHA, in a weight ratio of EPA:DHA of from 1.2 to 1.8;
b. An amount of +/-30% of 18.75 mg of hydroxytyrosol; and
c. An amount of +/-30% of 60 mg of curcumin.

5. The composition of any of claims 1-4, wherein the amounts defined therein as oscillating between +/-30%, are further defined as oscillating between +/-20%.

6. The composition of any of claims 1-4, wherein the amounts defined therein as oscillating between +/-30%, are further defined as oscillating between +/-5%.

7. The composition of claim 1, wherein said composition comprises each of elements a) to c) in the following amounts:
a. An amount of about 243.8 mg of Omega-3 polyunsaturated fatty acids (PUFAs) EPA and DHA, in a weight ratio of EPA:DHA of from 1.2 to 1.8;
b. An amount of about 37.5 mg of hydroxytyrosol; and
c. An amount of about 40 mg of curcumin.

8. The composition of any of claims 1-7 for use in a method of reducing C-reactive protein (CRP) in plasma, wherein this composition is administered orally.

9. The composition of claim 1 for use in a method of reducing C-reactive protein (CRP) in plasma, wherein said composition is administered in one or more daily dosages so that the daily amount of each of the three components is +/-30% of 731.4 mg of Omega-3 polyunsaturated fatty acids (PUFAs) EPA and DHA, +/-30% of 37.5 mg of hydroxytyrosol and +/-30% of 120 mg of curcumin, and wherein said composition is administered orally.

10. The composition of any of claims 1-7 for use in a method of increasing the overall survival rate of breast cancer patients diagnosed with said disease, wherein said composition is administered in one or more daily dosages so that the daily amount of each of the three components is +/-30% of 731.4 mg of Omega-3 polyunsaturated fatty acids (PUFAs) EPA and DHA, +/-30% of 37.5 mg of hydroxytyrosol and +/-30% of 120 mg of curcumin, and wherein said composition is administered orally.

## Patentansprüche

1. Zusammensetzung, wobei die genannte Zusammensetzung jedes der Elemente a) bis c) in den folgenden Mengen umfasst:
a. eine Menge von +/-30% von 243,8 mg bis 731,4 mg der Omega-3-mehrfach ungesättigten Fettsäuren (PUFAs) EPA und DHA, in einem Gewichtsverhältnis von EPA:DHA von 1,2 bis 1,8;
b. eine Menge von +/-30% von 12,5 mg bis 37,5 mg Hydroxytyrosol; und
c. eine Menge von +/-30% von 40 mg bis 120 mg Kurkumin.

2. Zusammensetzung nach Anspruch 1, wobei die genannte Zusammensetzung jedes der Elemente a) bis c) in den folgenden Mengen umfasst:
a. eine Menge von +/-30% von 731,4 mg der Omega-3-mehrfach ungesättigten Fettsäuren (PUFAs) EPA und DHA, in einem Gewichtsverhältnis von EPA:DHAvon 1,2 bis 1,8;
b. eine Menge von +/-30% von 37,5 mg Hydroxytyrosol; und
c. eine Menge von +/-30% von 120 mg Kurkumin.

3. Zusammensetzung nach Anspruch 1, wobei die genannte Zusammensetzung jedes der Elemente a) bis c) in den folgenden Mengen umfasst:
a. eine Menge von +/-30% von 243,8 mg der Omega-3-mehrfach ungesättigten Fettsäuren (PUFAs) EPA und DHA, in einem Gewichtsverhältnis von EPA:DHAvon 1,2 bis 1,8;
b. eine Menge von +/-30% von 12,5 mg Hydroxytyrosol; und
c. eine Menge von +/-30% von 40 mg Kurkumin.

4. Zusammensetzung nach Anspruch 1, wobei die genannte Zusammensetzung jedes der Elemente a) bis c) in den folgenden Mengen umfasst:
a. eine Menge von +/-30% von 365,7 mg der Omega-3-mehrfach ungesättigten Fettsäuren (PUFAs) EPA und DHA, in einem Gewichtsverhältnis von EPA:DHAvon 1,2 bis 1,8;
b. eine Menge von +/-30% von 18,75 mg Hydroxytyrosol; und
c. eine Menge von +/-30% von 60 mg Kurkumin.

5. Zusammensetzung nach einem der Ansprüche 1-4, wobei die darin als zwischen +/-30% schwankend definierten Mengen zusätzlich als zwischen +/-20% schwankend definiert werden.

6. Zusammensetzung nach einem der Ansprüche 1-4, wobei die darin als zwischen +/-30% schwankend definierten Mengen zusätzlich als zwischen +/-5% schwankend definiert werden.

7. Zusammensetzung nach Anspruch 1, wobei die genannte Zusammensetzung jedes der Elemente a) bis c) in den folgenden Mengen umfasst:
a. eine Menge von etwa 243,8 mg der Omega-3-mehrfach ungesättigten Fettsäuren (PUFAs) EPA und DHA, in einem Gewichtsverhältnis von EPA:DHAvon 1,2 bis 1,8;
b. eine Menge von etwa 37,5 mg Hydroxytyrosol; und
c. eine Menge von etwa 40 mg Kurkumin.

8. Zusammensetzung nach einem der Ansprüche 1-7, für deren Verwendung in einem Verfahren zur Verringerung des C-reaktiven Proteins (CRP) in Plasma, wobei diese Zusammensetzung oral verabreicht wird.

9. Zusammensetzung nach Anspruch 1, für deren Verwendung in einem Verfahren zur Verringerung des C-reaktiven Proteins (CRP) in Plasma, wobei die genannte Zusammensetzung in einer oder mehreren Tagesdosen verabreicht wird, sodass die tägliche Menge jedes der drei Bestandteile +/-30% von 731,4 mg der Omega-3-mehrfach ungesättigten Fettsäuren (PUFAs) EPA und DHA, +/-30% von 37,5 mg Hydroxytyrosol und +/-30% von 120 mg Kurkumin ist, und wobei die genannte Zusammensetzung oral verabreicht wird.

10. Zusammensetzung nach einem der Ansprüche 1-7, für deren Verwendung in einem Verfahren zur Erhöhung der Gesamtüberlebensrate von Brustkrebspatienten, welche mit der genannten Krankheit diagnostiziert wurden, wobei die genannte Zusammensetzung in einer oder mehreren Tagesdosen verabreicht wird, sodass die tägliche Menge jedes der drei Bestandteile +/-30% von 731,4 mg der Omega-3-mehrfach ungesättigten Fettsäuren (PUFAs) EPA und DHA, +/-30% von 37,5 mg Hydroxytyrosol und +/-30% von 120 mg Kurkumin ist, und wobei die genannte Zusammensetzung oral verabreicht wird.

## Revendications

1. Composition dans laquelle ladite composition comprend chacun des éléments a) à c) dans les quantités suivantes :
a. une quantité de +/-30% de 243,8 mg à 731,4 mg d'acides gras polyinsaturés oméga-3 (AGPI) EPA et DHA, dans un rapport en poids EPA:DHA de 1,2 à 1,8 ;
b. une quantité de +/-30% de 12,5 mg à 37,5 mg d'hydroxytyrosol ; et
c. une quantité de +/-30% de 40 mg à 120 mg de curcumine.

2. Composition selon la revendication 1, dans laquelle ladite composition comprend chacun des éléments a) à c) dans les quantités suivantes :
a. une quantité de +/-30% de 731,4 mg d'acides gras polyinsaturés oméga-3 (AGPI) EPA et DHA, dans un rapport en poids EPA:DHA de 1,2 à 1,8 ;
b. une quantité de +/-30% de 37,5 mg d'hydroxytyrosol ; et
c. une quantité de +/-30% de 120 mg de curcumine.

3. Composition selon la revendication 1, dans laquelle ladite composition comprend chacun des éléments a) à c) dans les quantités suivantes :
a. une quantité de +/-30% de 243,8 mg d'acides gras polyinsaturés oméga-3 (AGPI) EPA et DHA, dans un rapport en poids EPA:DHA de 1,2 à 1,8 ;
b. une quantité de +/-30% de 12,5 mg d'hydroxytyrosol ; et
c. une quantité de +/-30% de 40 mg de curcumine.

4. Composition selon la revendication 1, dans laquelle ladite composition comprend chacun des éléments a) à c) dans les quantités suivantes :
a. une quantité de +/-30% de 365,7 mg d'acides gras polyinsaturés oméga-3 (AGPI) EPA et DHA, dans un rapport en poids EPA:DHA de 1,2 à 1,8 ;
b. une quantité de +/-30% de 18,75 mg d'hydroxytyrosol ; et
c. une quantité de +/-30% de 60 mg de curcumine.

5. Composition selon l'une quelconque des revendications 1-4, dans laquelle les quantités définies dans celles-ci comme oscillant entre +/-30% sont en outre définies comme oscillant entre +/-20%.

6. Composition selon l'une quelconque des revendications 1-4, dans laquelle les quantités définies dans celles-ci comme oscillant entre +/-30% sont en outre définies comme oscillant entre +/-5%.

7. Composition selon la revendication 1, dans laquelle ladite composition comprend chacun des éléments a) à c) dans les quantités suivantes :
a. une quantité d'environ 243,8 mg d'acides gras polyinsaturés oméga-3 (AGPI) EPA et DHA, dans un rapport en poids EPA:DHA de 1,2 à 1,8 ;
b. une quantité d'environ 37,5 mg d'hydroxytyrosol ; et
c. une quantité d'environ 40 mg de curcumine.

8. Composition selon l'une quelconque des revendications 1-7, pour son utilisation dans un procédé de réduction de protéine C-réactive (CRP) dans le plasma, dans laquelle cette composition est administrée par voie orale.

9. Composition selon la revendication 1, pour son utilisation dans un procédé de réduction de protéine C-réactive (CRP) dans le plasma, dans laquelle ladite composition est administrée en une ou plus doses journalières de sorte que la quantité journalière de chacun des trois composants est de +/- 30% de 731,4 mg d'acides gras polyinsaturés oméga-3 (AGPI) EPA et DHA, +/-30% de 37,5 mg d'hydroxytyrosol et +/-30% de 120 mg de curcumine, et dans laquelle ladite composition est administrée par voie orale.

10. Composition selon l'une quelconque des revendications 1-7, pour son utilisation dans un procédé d'augmentation du taux de survie global de patientes atteintes d'un cancer du sein diagnostiquées avec ladite maladie, dans laquelle ladite composition est administrée en une ou plusieurs doses journalières de sorte que la quantité journalière de chacun des trois composants est de +/-30% de 731,4 mg d'acides gras polyinsaturés oméga-3 (AGPI) EPA et DHA, +/-30% de 37,5 mg d'hydroxytyrosol et +/-30% de 120 mg de curcumine, et dans laquelle ladite composition est administrée par voie orale.
